(19) **Europäisches Patentamt / European Patent Office / Office européen des brevets**

(11) **EP 3 202 279 A1**

(12) # EUROPEAN PATENT APPLICATION
published in accordance with Art. 153(4) EPC

(43) Date of publication:
**09.08.2017 Bulletin 2017/32**

(21) Application number: **15847643.2**

(22) Date of filing: **30.09.2015**

(51) Int Cl.:
**A45D 44/22** (2006.01)   **A61K 8/02** (2006.01)
**A61K 8/26** (2006.01)   **A61K 8/73** (2006.01)
**A61K 8/81** (2006.01)   **A61Q 19/00** (2006.01)

(86) International application number:
**PCT/JP2015/077816**

(87) International publication number:
**WO 2016/052651 (07.04.2016 Gazette 2016/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **30.09.2014   JP 2014201743**

(71) Applicant: **Sekisui Plastics Co., Ltd.**
**Osaka-shi**
**Osaka 530-8565 (JP)**

(72) Inventor: **HATORI, Takaaki**
**Inashiki-gun**
**Ibaraki 300-0421 (JP)**

(74) Representative: **Hoffmann Eitle**
**Patent- und Rechtsanwälte PartmbB**
**Arabellastraße 30**
**81925 München (DE)**

(54) **COSMETIC GEL SHEET**

(57)   A cosmetic gel sheet which shows a remarkable adhesion property, moisture-retaining property, and skin-firming feel is provided. Each of the cosmetic gel sheets (10L, 10R) has a base material made of a nonwoven or woven fabric, and a gel layer containing a cosmetic composition. In this cosmetic gel sheet, one direction orthogonal to a thickness direction is assumed to be a direction $D_A$, and a direction orthogonal to both of the thickness direction and the one direction is assumed to be a direction $D_B$. An elongation percentage in the direction $D_A$ of the cosmetic gel sheet is 10 to 40%. An elastic recovery percentage of elongation, measured after the cosmetic gel sheet is stretched in the direction $D_A$ by 50% and kept stretched at 23°C, 55% RH for 10 minutes, is 5% or less. An elongation percentage in the direction of the cosmetic gel sheet $D_B$ is 2% or less.

FIG.1

EP 3 202 279 A1

**Description**

[Technical Field]

The present invention relates to a cosmetic gel sheet which shows a remarkable adhesion property, moisture-retaining property, and skin-firming feel, and which is suitable as a cosmetic gel sheet for facial application.

[Background Art]

As cosmetic gel sheets for skin care, cosmetic packs in each of which a nonwoven fabric and a gel layer containing a cosmetic composition is laminated are known. In use, these cosmetic packs are applied to the facial skin.
Conventionally, in order to improve adhesion property or a facelift effect of such cosmetic packs, a cosmetic mask which is an integrally laminated product of a non-elastomeric fiber layer and an elastomer layer and which is stretchable in vertical directions of the face has been provided (see PTL 1).
Conventional cosmetic packs also include a cosmetic sheet for facial application, intended to improve fitting property, adhesion property and handlability and to avoid discomfort in use (e.g. excessive tautness). Such a cosmetic sheet is composed of an elastic ground fabric and a tackifier composition containing polyacrylic acid, sodium polyacrylate, and purified water. As measured with a 50-mm-wide strip-shaped specimen under a load of 1 N, this cosmetic sheet shows elongation percentages of 7.3 to 12% in vertical directions and 17 to 20% in crosswise directions, as applied to the face (see Examples in
PTL 2).

[Citation List]

[Patent Literature]

[PTL 1] JP 2013-128743 A
[PTL 2] JP 2000-128732 A

[Summary of the Invention]

[Technical Problem]

The cosmetic mask disclosed in PTL 1 is applied to the face by being stretched downwardly of the face. Owing to the action of the elastomer layer whose elastic recovery percentage of elongation is high, the cosmetic mask generates a large contractile force in an upward direction of the face, pulls the facial skin upwardly, and thereby provides a facelift effect. Since the cosmetic mask disclosed in PTL 1 strongly pulls the facial skin upwardly and physically makes the skin stiff (the skin gets too taut), this mask fails to provide a comfortable feel in use. In other words, the cosmetic mask disclosed in PTL 1 performs poorly in smoothing wrinkles and realizing inherent natural firmness of the skin (hereinafter mentioned as "skin-firming feel"). Besides, the cosmetic mask disclosed in PTL 1, which lacks a comfortable feel in use, cannot supply sufficient moisture to the skin and thus shows a poor moisture-retaining property.
The cosmetic sheet disclosed in PTL 2, which shows relatively high elongation percentages of 7.3% or higher in both the vertical directions and the crosswise directions, has a poor shape-retaining property (i.e. less "resilient"). When applied to the skin, this cosmetic sheet deforms badly and cannot provide a conformtable feel in use. In other words, the cosmetic sheet disclosed in PTL 2 performs poorly in smoothing wrinkles and realizing inherent natural firmness of the skin (skin-firming feel). Besides, the cosmetic sheet disclosed in PTL 2, which lacks a comfortable feel in use, cannot supply sufficient moisture to the skin and thus shows a poor moisture-retaining property.
The present invention has been made in view of these circumstances, and aims to provide a cosmetic gel sheet which shows a remarkable adhesion property, moisture-retaining property, and skin-firming feel.

[Solution to Problem]

A cosmetic gel sheet according to the present invention includes a base material made of a nonwoven or woven fabric, and a gel layer containing a cosmetic composition. In this cosmetic gel sheet, one direction orthogonal to a thickness direction is assumed to be a direction $D_A$, and a direction orthogonal to both of the thickness direction and the one direction is assumed to be a direction $D_B$. An elongation percentage in the direction $D_A$ of the cosmetic gel sheet is 10 to 40%. An elastic recovery percentage of elongation, measured after the cosmetic gel sheet is stretched in the direction $D_A$ by 50% and kept stretched at 23°C, 55% RH for 10 minutes, is 5% or less. An elongation percentage in the direction $D_B$ of the cosmetic gel sheet is 2% or less.

Since the elongation percentage in the direction $D_A$ is 10 to 40%, the thus configured cosmetic gel sheet can be applied to the skin along the skin contour by being stretched moderately in the direction $D_A$, and thus exhibits a remarkable adhesion property. Further, since the elastic recovery percentage of elongation, measured after the cosmetic gel sheet is stretched in the direction $D_A$ by 50% and kept stretched at 23°C, 55% RH for 10 minutes, is 5% or less, the thus configured cosmetic gel sheet which has been applied to the face by being stretched in the direction $D_A$ is prevented from shrinking to its original state. Hence, the cosmetic gel sheet can keep giving natural firmness to the skin and smoothing out the wrinkles, and can thereby provide a remarkable skin-firming feel and moisture-retaining property. Furthermore, since the elongation percentage in the direction $D_B$ is 2% or less, the thus configured cosmetic gel sheet which has been applied to the face by being stretched in the direction $D_A$ is less likely to deform and provides a remarkable feel in use. As a result, the cosmetic gel sheet can keep giving natural firmness to the skin and smoothing out the wrinkles, and can thereby provide a remarkable skin-firming feel and moisture-retaining property.

[Advantageous Effects of Invention]

The present invention can provide a cosmetic gel sheet which shows a remarkable adhesion property, moisture-retaining property, and skin-firming feel.

[Brief Description of the Drawings]

Fig. 1 is a plan view showing an embodiment of the cosmetic gel sheet according to the present invention.
Fig. 2 is a plan view showing another embodiment of the cosmetic gel sheet according to the present invention.
Fig. 3 is a plan view showing still another embodiment of the cosmetic gel sheet according to the present invention.
Fig. 4 is a plan view showing yet another embodiment of the cosmetic gel sheet according to the present invention.

[Description of Embodiments]

Hereinafter, the present invention is described in more detail.

[Cosmetic gel sheet]

A cosmetic gel sheet according to the present invention includes a base material made of a nonwoven or woven fabric, and a gel layer containing a cosmetic composition. In this cosmetic gel sheet, one direction orthogonal to a thickness direction is assumed to be a direction $D_A$, and a direction orthogonal to both of the thickness direction and the one direction is assumed to be a direction $D_B$. An elongation percentage in the direction $D_A$ of the cosmetic gel sheet is 10 to 40%. An elastic recovery percentage of elongation, measured after the cosmetic gel sheet is stretched in the direction $D_A$ by 50% and kept stretched at 23°C, 55% RH for 10 minutes (hereinafter simply called "elastic recovery percentage of elongation in the direction $D_A$"), is 5% or less. An elongation percentage in the direction $D_B$ of the cosmetic gel sheet is 2% or less. In use, the cosmetic gel sheet is applied to the skin, with the gel layer surface being in contact with the skin. Since the elongation percentage in the direction $D_A$ is as high as 10 to 40%, the cosmetic gel sheet can be applied in tight contact with the skin by being stretched in the direction $D_A$.

In the cosmetic gel sheet, the elongation percentage in the direction $D_A$ may be from 10 to 40%, and preferably from 15 to 35%. If the elongation percentage in the direction $D_A$ is less than 10%, the cosmetic gel sheet has a poor adhesion property because the cosmetic gel sheet cannot stretch well in the direction $D_A$ and cannot follow the skin contour sufficiently. If the elongation percentage in the direction $D_A$ exceeds 40%, the cosmetic gel sheet has such a poor shape-retaining property that it deforms badly when applied to the skin, thus deteriorating the skin-firming feel and the moisture-retaining property. The elongation percentage in the direction $D_A$ shall be measured by a measurement method to be described in Examples.

In the cosmetic gel sheet, the elastic recovery percentage of elongation in the direction $D_A$ may be 5% or less, and preferably 3% or less. If the elastic recovery percentage of elongation in the direction $D_A$ exceeds 5%, the cosmetic gel sheet which has been applied to the skin by being stretched in the direction $D_A$ shrinks so greatly as to physically make the skin stiff (the skin gets too taut), thus deteriorating the skin-firming feel and the moisture-retaining property. The elastic recovery percentage of elongation in the direction $D_A$ shall be measured by a measurement method to be described in Examples.

In the cosmetic gel sheet, the elongation percentage in the direction $D_B$ may be 2% or less, and preferably 1.5% or less. If the elongation percentage in the direction $D_B$ exceeds 2%, the cosmetic gel sheet has such a poor shape-retaining property that it deforms badly when applied to the skin by being stretched in the direction $D_A$. Eventually, the cosmetic gel sheet gives a poor skin-firming feel and a poor moisture-retaining property. The elongation percentage in the direction $D_B$ shall be measured by a measurement method to be described in Examples.

Preferably, the cosmetic gel sheet has a vertical adhesion strength of 0.020 to 5.00 N/12 mmφ, and more preferably 0.020 to 2.00 N/12 mmφ. If the vertical adhesion strength is less than 0.020 N/12 mmφ, the cosmetic gel sheet which has been applied to the skin may not adhere to the skin tightly enough. If the vertical adhesion strength exceeds 5.00 N/12 mmφ, the cosmetic gel sheet which has been applied to the skin may physically make the skin stiff (the skin gets too taut), thus deteriorating the skin-firming feel and handlability of the cosmetic gel sheet itself. The vertical adhesion strength shall be measured by a measurement method to be described in Examples.

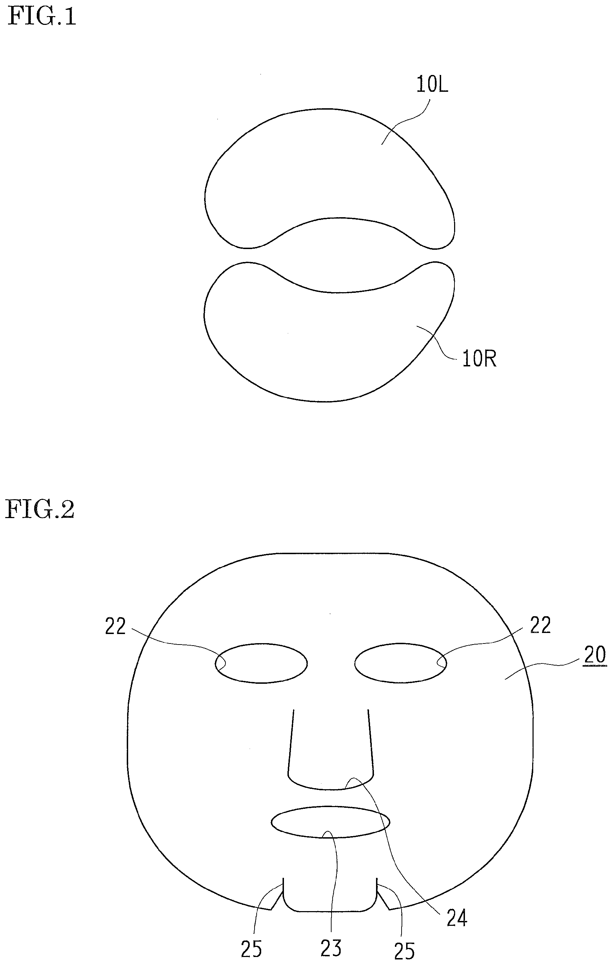

The cosmetic gel sheet according to the present invention is suitable as a cosmetic gel sheet for application to a face. As the cosmetic gel sheet for facial application, some exemplary shapes are described and illustrated in following embodiments and the attached drawings. Fig. 1 is a plan view showing a pair of undereye cosmetic gel sheets, as an embodiment of the cosmetic gel sheet according to the present invention.

A pair of cosmetic gel sheets 10L, 10R shown in Fig. 1 are designed for application under the left and right eyes, respectively. The lower part of the cosmetic gel sheet 10L, as seen in Fig. 1, has a substantially arc-like outline conforming to the left lower eyelid. In use, the cosmetic gel sheet 10L is applied to the skin just under the left eye, with this lower outline following the left lower eyelid. The upper part of the cosmetic gel sheet 10R, as seen in Fig. 1, has a substantially arc-like outline conforming to the right lower eyelid. In use, the cosmetic gel sheet 10R is applied to the skin just under the right eye, with this upper outline following the right lower eyelid. In the cosmetic gel sheets 10L, 10R, the direction $D_A$ is preferably a horizontal direction (a crosswise direction in Fig. 1), for further improvement in the skin-firming feel.

Shapes of the cosmetic gel sheet for facial application are not limited to those shown in Fig. 1, but may be designed to cover the entire face as shown in Fig. 2. A cosmetic gel sheet 20 shown in Fig. 2, designed for the entire face, is a substantially circular sheet corresponding to a size of the entire face. The cosmetic gel sheet 20 is provided with two holes 22 for the eyes, a hole 23 for the mouth, a slit 24 for the nose, and slits 25 for the chin. The holes 22, 23 and the slits 24, 25 are positioned to allow the cosmetic gel sheet 20 to be applied to the entire face along the curved contour of the face. Also in the case of the cosmetic gel sheet 20, the direction $D_A$ is preferably a horizontal direction (a crosswise direction in Fig. 2), for further improvement in the skin-firming feel.

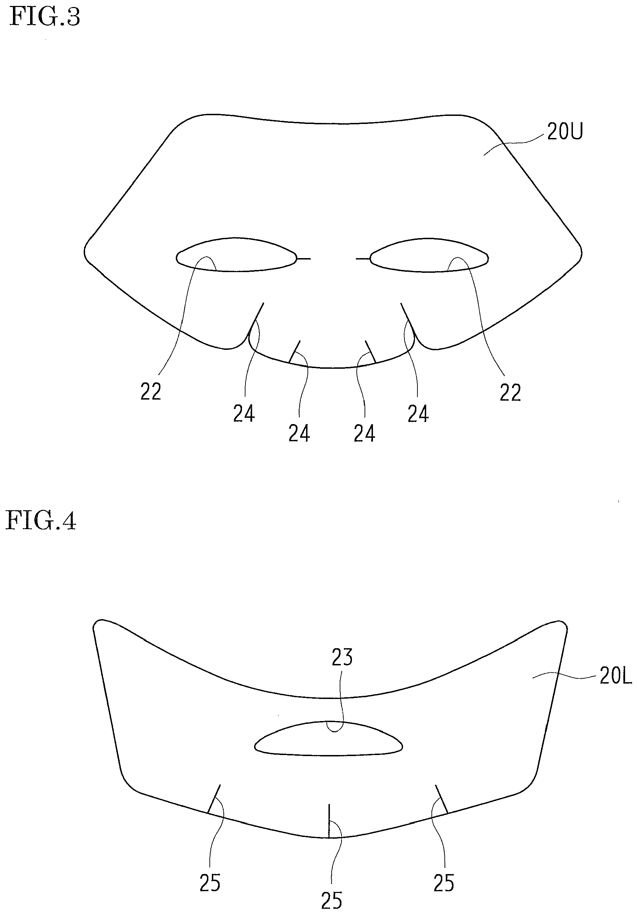

Further, the cosmetic gel sheet for facial application may be a cosmetic gel sheet 20U designed to cover the upper half of a face as shown in Fig. 3, or may be a cosmetic gel sheet 20L designed to cover the lower half of a face as shown in Fig. 4. The cosmetic gel sheet 20U having a size corresponding to the upper half of the face and the cosmetic gel sheet 20L having a size corresponding to the lower half of the face may be used separately, or may be used in combination to cover the entire face. In this context, the upper half of the face includes at least a forehead area, excluding a chin area and cheek areas, and the lower half of the face includes the chin area and the cheek areas, excluding the forehead area. In Figs. 3 and 4, description of the same elements as those mentioned in Fig. 2 is omitted by assigning the same reference signs. Also in the case of the cosmetic gel sheets 20U and 20L, the direction $D_A$ is preferably a horizontal direction (a crosswise direction in Figs. 3 and 4), for further improvement in the skin-firming feel.

The above description is directed to the cosmetic gel sheets for facial application. Additionally, the cosmetic gel sheet according to the present invention may be applied to the other part of the body than the face, for example, to the back of the hand.

[Base material]

Regarding the woven or nonwoven fabric which constitutes the base material, a mass per unit area of the fabric is not particularly limited as far as the predetermined physical properties of the cosmetic gel sheet according to the present invention can be satisfied. Having said that, the mass per unit area of the fabric is preferably from 40 to 150 $g/m^2$, and more preferably from 50 to 130 $g/m^2$. If the mass per unit area of the woven or nonwoven fabric is less than 40 $g/m^2$, the gel may bleed through the fabric (the gel constituting the gel layer permeates through the woven or nonwoven fabric to the other side of the base material, opposite to the gel layer side). When the gel bleeds through, the base material is practically buried in the gel and becomes less elastic, so that the elongation percentage in the direction $D_A$ may decrease. On the other hand, if the mass per unit area of the woven or nonwoven fabric exceeds 150 $g/m^2$, the base material may be so hard as to lose the skin adhesion property. The fineness of the fiber constituting the woven or nonwoven fabric is not particularly limited, but is preferably from 0.5 to 3.0 dtex. The thickness of the woven or nonwoven fabric is preferably from 0.20 mm to 0.80 mm.

The fiber constituting the woven or nonwoven fabric is not particularly limited as far as the predetermined physical properties of the cosmetic gel sheet according to the present invention can be satisfied. The fiber may be whichever of a natural fiber or a chemical fiber. Examples of the natural fiber include natural cellulose fibers such as cotton, pulp, and hemp. Examples of the chemical fiber include synthetic fibers, semisynthetic fibers produced by reforming natural fibers, regenerated cellulose fibers produced by chemically extracting vegetable celluloses and regenerating the extracted vegetable celluloses into fibers, purified cellulose fibers produced by purifying vegetable celluloses, and the like. Examples of the synthetic fibers include polyester fibers such as polyethylene terephthalate (hereinafter abbreviated as "PET"), polypropylene fibers, nylon fibers, and the like. Examples of the regenerated cellulose fibers include cupro (cuprammonium rayon), viscose rayon, polynosic rayon, and the like. Materials for the regenerated cellulose fibers may be, for example, cotton linter, pulp, and the like. Examples of the purified cellulose fibers include lyocell (TENCEL®).

The fiber constituting the woven or nonwoven fabric may be produced with use of whichever of chopped fibers or continuous fibers. Having said that, continuous fibers are preferable, and continuous filaments are more preferable. By using preferable fibers, the cosmetic gel sheet according to the present invention can easily satisfy its predetermined physical properties. Examples of the continuous filaments include regenerated cellulose continuous filaments such as cupro continuous filaments. The woven or nonwoven fabric may be composed of a combination of two or more fibers. For example, the woven or nonwoven fabric may be a woven or nonwoven fabric made of a blended yarn of a synthetic fiber with a natural fiber, a semisynthetic fiber, a regenerated cellulose fiber, or a purified cellulose fiber, or may be a nonwoven fabric produced by blending a synthetic fiber with a natural fiber, a semisynthetic fiber, a regenerated cellulose fiber, or a purified cellulose fiber.

Specific examples of the nonwoven fabric include a needlepunch nonwoven, a spunbonded nonwoven, a spunlace nonwoven, a crimped nonwoven made of a crimped yarn, and the like.

[Gel layer]

To form the gel layer, a gel composition for forming the gel layer is applied to one surface of the base material and cured until the gel composition turns into the gel state. Usually, a part of the gel composition permeates into the base material, cures therein, and is thereby integrated with the base material to form a middle layer. The gel constituting the gel layer may be prepared by blending at least either of a network polymer or a thickening agent (a mucilaginous agent) with water and a cosmetic composition.

Preferably, the gel layer is a hydrogel layer containing 25 to 95% by weight of water. If the water content is less than 25% by weight, the cosmetic gel sheet which has been applied to the skin cannot provide sufficient moisture. If the water content exceeds 95% by weight, the gel strength decreases so much as to deteriorate handlability.

The network polymer is not particularly limited as far as having an affinity for water and being conventionally employed in dermatologic agents such as cosmetics and pharmaceutical agents. The network polymer may be chosen from a variety of synthetic polymers and natural polymers each having a network. Among them, a particularly preferable network polymer is a synthetic polymer having a network, in which a polymer skeleton is a polymer made from a polymerizable unsaturated monomer having an anionic functional group such as a carboxyl group or a sulfonic acid group, or a salt (e.g. sodium salt, potassium salt, triethanolamine salt) of this polymer, and which is obtained by adding the polymer skeleton and a crosslinking agent to the gel composition and effecting a reaction with the crosslinking agent.

Examples of the polymer made from a polymerizable unsaturated monomer having a carboxyl group or a salt of this polymer include polyacrylic acid or a salt thereof, polymethacrylic acid or a salt thereof, and the like. Examples of the polymer made from a polymerizable unsaturated monomer having a sulfonic acid group or a salt of this polymer include poly(t-butylacrylamidosulfonic acid) or a salt thereof.

Examples of the crosslinking agent include aluminum hydroxide, potassium alum, aluminum sulfate, aluminum glycinate, aluminum acetate, aluminum oxide, aluminum metasilicate, magnesium chloride, calcium hydroxide, calcium carbonate, magnesium aluminometasilicate, polyethyleneimine, polyethylene glycol diglycidyl ether, glycerol triglycidyl ether, triglycidyl isocyanurate, and the like.

To the gel composition in which the crosslinking agent has been blended, it is also possible to add a crosslinking accelerator which promotes a crosslinking reaction by adjusting the pH of the gel composition to an optimum pH for the action of the crosslinking agent. Examples of the crosslinking agent include organic acids such as tartaric acid, lactic acid, citric acid, and glycolic acid, and inorganic acids such as hydrochloric acid. The pH of the gel composition after the addition of the crosslinking accelerator is preferably from 4 to 6, and more preferably from 4 to 5.

The network polymer may be a copolymer made from a (meth)acrylamide and/or a (meth)acrylamide derivative and a crosslinkable monomer. The (meth)acrylamide and/or the (meth)acrylamide derivative is not particularly limited unless being a crosslinkable monomer, and may be, for example, non-electrolytic (meth)acrylamides or derivatives thereof such as tert-butylacrylamidosulfonic acid (TBAS), tert-butylacrylamidosulfonate, N,N-dimethylaminoethyl acrylamide (DMAEAA) hydrochloride, N,N-dimethylaminopropyl acrylamide (DMAPAA) hydrochloride, (meth)acrylamide, N-methyl (meth)acrylamide, N-ethyl (meth)acrylamide, N-propyl (meth)acrylamide, N,N-dimethyl (meth)acrylamide, N,N-diethyl (meth)acrylamide, acryloylmorpholine, and the like. These compounds may be used alone or in combination.

The crosslinkable monomer is not particularly limited, but preferably has two or more polymerizable double bonds in a molecule. Examples of the crosslinkable monomer include N,N'-methylenebis(meth)acrylamide, N,N'-ethylenebis(meth)acrylamide, (poly)ethylene glycol di(meth)acrylate, (poly)propylene glycol di(meth)acrylate, 1,9-nonanediol di(meth)acrylate, glycerol di(meth)acrylate, glycerol tri(meth)acrylate, tetraallyloxyethane, diallylammonium chloride, and the like. These compounds may be used alone or in combination.

Examples of the thickening agent include hydroxypropyl guar gum, agar, gelatin, carrageenan, xanthan gum, celluloses (carboxymethylcellulose (CMC), methylcellulose (MC), hydroxypropylcellulose (HPC), etc.), polyacrylic acid or a salt thereof, polymethacrylic acid or a salt thereof, polyvinyl alcohol, polyvinylpyrrolidone, alginic acid or a salt thereof, polyacrylamide, a copolymer made from monomers which form two or more of these polymers, or a copolymer made from a monomer which forms any one of these polymers and a different monomer, and the like.

Examples of the cosmetic composition to be held in the gel layer include bleed accelerators, moisturizers, cooling agents, warming agents, plant/animal extracts (medicinal ingredients), biopolymers, oily ingredients, anti-inflammatory agents, vitamins, active ingredients, antioxidants, blood circulation promoting agents, wound healing agents, and other components. In addition to the cosmetic composition, the gel layer may hold a conventional ingredient for dermatologic agents such as cosmetics and pharmaceutical agents. Examples of such additional ingredient include fragrances, colorants, stabilizers, antioxidants, UV agents, tackifiers, chelating agents, antiseptics (e.g. phenoxyethanol), antibacterial agents, emulsifiers, electrolytes, and the like.

The electrolytes are not particularly limited. Examples of the electrolytes include: alkali metal halides such as sodium halides (e.g. sodium chloride), potassium halides, magnesium halides, calcium halides, alkaline earth metal halides, and other metal halides; salts formed from metals and inorganic acids such as hypochlorous acid, chlorous acid, chloric acid, perchloric acid, sulfuric acid, nitric acid, and phosphoric acid, or ammonium salts formed from these inorganic acids; complex salts; salts formed from metals and monovalent organic carboxylic acids such as acetic acid, benzoic acid, and lactic acid, or ammonium salts formed from these monovalent organic carboxylic acids; salts formed from one or more metals or ammonium and multivalent carboxylic acids such as phthalic acid, succinic acid, adipic acid, citric acid, and tartaric acid; salts formed from metals and organic acids such as sulfonic acid and amino acid, or ammonium salts formed from these organic acids; salts formed from metals and polyacids such as poly(meth)acrylic acid, polyvinylsulfonic acid, poly(tert-butylacrylamidosulfonic) acid, polyallylamine, and polyethyleneimine, or ammonium salts (polyelectrolyte salts) formed from these polyacids. These compounds may be used alone or in combination.

The bleed accelerators are water-insoluble components which are easily expelled from the gel layer without being integrated with the gel layer. Specific examples of the bleed accelerators include polyethylene glycol 1000, polyethylene glycol 1500, polyethylene glycol 2000, polyethylene glycol 3000, and the like. For example, if the base material for the cosmetic gel sheet is a nonwoven fabric made of a PET fiber or the like, the bleed accelerator is added preferably in an amount of 1 to 2% by weight relative to the gel composition for constituting the gel layer.

Specific examples of the moisturizers include polyols such as glycerol, diglycerol, triglycerol, polyglycerol, 3-methyl-1,3-butanediol (isoprene glycol), 1,3-butylene glycol, 1,2-pentanediol, 1,2-hexanediol, propylene glycol, dipropylene glycol, polypropylene glycol, ethylene glycol, diethylene glycol, triethylene glycol, pentaerythritol, neopentyl glycol, maltitol, reduced sugar syrup, sucrose, lactitol, palatinit, erythritol, sorbitol, mannitol, xylitol, xylose, glucose, lactose, mannose, maltose, galactose, fructose, inositol, raffinose, trehalose, trimethylglycine, cyclodextrin, hyaluronic acid, and salts thereof, amino acids, urea, sodium pyrrolidone carboxylic acid, betaines, whey, olive squalene, and the like. These moisturizers may be used alone or in combination.

Specific examples of the cooling agents include menthol, menthone, camphor, pulegol, isopulegol, cineole, mentha oil, peppermint oil, spearmint oil, eucalyptus oil, 3-1-menthoxy propane-1,2-diol, N-alkyl-p-menthane-3-carboxamide, 3-1-menthoxy-2-methylpropane-1,2-diol, p-menthane-3,8-diol, 2-1-menthoxyethane-1-ol, 3-1-menthoxypropane-1-ol, 4-1-menthoxybutane-1-ol, menthyl 3-hydroxybutanoate, menthyl lactate, menthone glycerol ketal, 2-(2-1-menthyloxyethyl)ethanol, menthyl glyoxylate, N-methyl-2,2-isopropylmethyl-3-methylbutanamide, 2-pyrrolidone-5-carboxylic acid menthyl ester, monomenthyl succinate, alkali metal salts of monomenthyl succinate, alkaline earth metal salts of monomenthyl succinate, and the like. These cooling agents may be used alone or in combination.

Specific examples of the warming agents include vanillyl ethyl ether, vanillyl propyl ether, vanillin propylene glycol acetal, ethyl vanillin propylene glycol acetal, capsaicin, gingerol, vanillyl butyl ether, vanillyl butyl ether acetate, 4-(1-menthoxy methyl)-2-phenyl-1,3-dioxolane, 4-(1-menthoxy methyl)-2-(3',4'-dihydroxyphenyl)-1,3-dioxolane, 4-0-methoxy methyl)-2-(2'-hydroxy-3'-methoxy phenyl)-1,3-dioxolane, 4-(1-menthoxy-methyl)-2-(4'-methoxy phenyl)-1,3-dioxolane, 4-(1-menthoxy methyl)-2-(3',4'-methylenedioxyphenyl)-1,3-dioxolane, 4-(1-methoxymethyl)-2-(3'-methoxy-4'-hydroxyphenyl)-1, 3-dioxolane, capsicum oil, capsicum oleoresin, ginger oleoresin, nonylic acid vanillylamide, jambu oleoresin, zanthoxylum extract, sanshool-I, sanshool-II, amide of zanthoxylum, black pepper extract, chavicine, piperine, spilanthol, and the like. These warming agents may be used alone or in combination.

Specific examples of the plant/animal extracts include angelica extract, avocado extract, hydrangea extract, althaea extract, arnica extract, apricot extract, apricot kernel extract, fennel extract, turmeric extract, oolong tea extract, echinacea leaf extract, scutellaria root extract, phellodendron bark extract, barley extract, watercress extract, orange

6

extract, sea salt, hydrolyzed elastin, hydrolyzed wheat protein, hydrolyzed silk, chamomile extract, carrot extract, artemisia capillaris extract, glycyrrhiza (licorice) extract, karkade extract, kiwi extract, cinchona extract, cucumber extract, guanosine, sasa veitchii extract, walnut extract, grapefruit extract, clematis extract, yeast extract, burdock root extract, comfrey extract, collagen, vaccinium vitis-idaea (cowberry or lingonberry) extract, bupleurum root extract, umbilical extract, clary extract, saponaria extract, sasa extract, crataegus cuneate extract, shiitake mushroom extract, rehmannia root extract, lithospermum root extract, tilia japonica extract, filipendula extract, calamus rhizome extract, birch extract, horsetail extract, honeysuckle extract, ivy extract, crataegus oxyacantha extract, sambucus extract, yellow extract, peppermint extract, mallow extract, swertia herb extract, jujube extract, thyme extract, clove extract, imperata cylindrica extract, citrus unshiu peel extract, bitter orange peel extract, houttuynia extract, tomato extract, natto extract, ginseng extract, wild rose extract, hibiscus sabdariffa extract, ophiopogon tuber extract, parsley extract, honey, pellitory extract, isodonis extract, bisabolol, coltsfoot extract, butterbur extract, hoelen extract, butcher broom extract, grape extract, propolis, sponge gourd extract, peppermint extract, tilia europaea extract, hop extract, pine extract, horse chestnut extract, lysichiton camtschatcense (skunk cabbage) extract, mukurossi peel extract, peach extract, cornflower extract, eucalyptus extract, citrus junos (yuzu) extract, mugwort extract, lavender extract, apple extract, lettuce extract, lemon extract, Chinese milk vetch extract, rose extract, rosemary extract, Roman chamomile extract, royal jelly extract, astaxanthin, and the like. These plant/animal extracts may be used alone or in combination.

Specific examples of the biopolymers include deoxyribonucleic acid, sodium chondroitin sulfate, collagen, elastin, chitin, chitosan, hydrolyzed egg shell membrane, and the like. These biopolymers may be used alone or in combination.

Specific examples of the oily ingredients include: hydrocarbons such as sphingolipids, ceramides, cholesterol, cholesterol derivatives, phospholipids, silicone polymers, mineral oils, paraffins, liquid paraffins, isoparaffins, hydrogenated polydecene, and isohexadecane; esters such as trimethylolpropane tricaprylate/tricaprate, alkyl benzoates having 12 to 15 carbon atoms, ethylhexyl stearate, caprylic/capric acid triglyceryl, squalane, ethylhexyl cocoate, decyl oleate, decyl cocoate, ethyl oleate, isopropyl myristate, ethylhexyl perlagonate, pentaerythrityl tetracaprylate/tetracaprate, PPG-3 benzyl ether myristate, propylene glycol dicaprylate/dicaprate, ethylhexyl isostearate, and ethylhexyl palmitate; natural oils such as soybean oil, sunflower seed oil, jojoba oil, safflower oil, akahana (red flower) oil, rape seed oil, and derivatives thereof; and the like. These oily ingredients may be used alone or in combination.

Specific examples of the anti-inflammatory agents include ε-aminocaproic acid, glycyrrhizic acid, dipotassium glycyrrhizinate, 6-glycyrrhetic acid, lysozyme chloride, guaiazulene, hydrocortisone, and the like. These anti-inflammatory agents may be used alone or in combination.

Specific examples of the vitamins include vitamins A, $B^2$, $B^6$, D, E; calcium pantothenate; biotic," nicotinamide; ascorbic acid, ascorbic acid derivatives, or a salt thereof; and the like. Specific examples of the ascorbic acid, the ascorbic acid derivatives or a salt thereof include L-ascorbic acid, sodium L-ascorbate, potassium L-ascorbate, calcium L-ascorbate, L-ascorbyl phosphate, L-ascorbyl phosphate magnesium salt, L-ascorbyl sulfate, L-ascorbyl sulfate disodium salt, L-ascorbyl stearate, L-ascorbyl 2-glucoside, L-ascorbyl palmitate, L-ascorbyl tetraisopalmitate, and the like. These vitamins may be used alone or in combination.

Specific examples of the active ingredients include allantoin, diisopropylamine dichloroacetate, 4-(aminomethyl)cyclohexane carboxylic acid, and the like. These active ingredients may be used alone or in combination.

Specific examples of the antioxidants include tocopherol, carotenoid, flavonoid, tannin, lignan, saponin, and the like. These antioxidants may be used alone or in combination.

Specific examples of the blood circulation promoting agents include γ-orizanol, vitamin E derivatives, and the like. These blood circulation promoting agents may be used alone or in combination.

Specific examples of the wound healing agents are retinol, retinol derivatives, and the like. These wound healing agents may be used alone or in combination.

Specific examples of the other components include cepharanthine, capsicum tincture, hinokitiol, iodized garlic extract, pyridoxine hydrochloride, dl-α-tocopherol, dl-α-tocopherol acetate, nicotinic acid, nicotinic acid derivatives, calcium pantothenate, D-pantothenyl alcohol, acetylpantothenyl ethyl ether, biotin, allantoin, isopropyl methylphenol, estradiol, ethinylestradiol, carpronium chloride, benzalkonium chloride, diphenhydramine hydrochloride, Takanal, camphor, salicylic acid, nonylic acid vanillylamide, nonanoic acid vanillylamide, piroctone olamine, glyceryl pentadecanoate, mononitroguaiacol, resorcin, γ-aminobutyric acid, benzethonium chloride, mexiletine hydrochloride, auxin, female hormone, cantharides tincture, ciclosporin, hydrocortisone, polyoxyethylene sorbitan monostearate, analgesics, tranquilizers, antihypertensives, antibiotics, antihistamines, antibacterial substances, and the like. These components may be used alone or in combination.

The thickness of the gel layer (including the thickness of the middle layer) is preferably from 0.3 to 1.5 mm. If the thickness of the gel layer is less than 0.3 mm, the skin adhesion property and the gel feeling may not be satisfactory. If the thickness of the gel layer exceeds 1.5 mm, the cosmetic gel sheet is so heavy that the cosmetic gel sheet may peel off from the skin by its own weight. In addition, if the thickness of the gel layer exceeds 1.5 mm, a user may feel uncomfortable while the cosmetic gel sheet is applied to the skin.

The thickness of the gel layer (including the thickness of the middle layer) is preferably from 10 to 90%, and more preferably from 35 to 80%, relative to the total thickness of the cosmetic gel sheet. If the thickness of the gel layer is less than 10% relative to the total thickness of the cosmetic gel sheet, the skin adhesion property and the gel feeling may not be satisfactory. If the thickness of the gel layer exceeds 90% relative to the total thickness of the cosmetic gel sheet, there are some concerns: the gel layer may deteriorate the physical properties of the cosmetic gel sheet such as the elongation percentage of the base material; the gel may bleed through; and the gel which has bled through during the production of the cosmetic gel sheet may contaminate the production line.

To form the gel layer, the gel composition for forming the gel layer is applied to the base material and cured thereon. The gel composition which contains the polymer skeleton and the crosslinking agent effects a quick crosslinking reaction by the crosslinking agent in the gel composition, and thereby cures quickly. Hence, the gel composition should be mixed thoroughly and applied to the base material within a predetermined time after the crosslinking reaction is initiated by the crosslinking agent. The ratio of the amount of gel permeating into the base material (the gel constituting the middle layer) to the amount of gel curing on the surface of the base material (a part of the gel layer excluding the middle layer) is decided by at which stage of the crosslinking reaction the gel composition should be applied. Accoringly, at the start of the crosslinking reaction (in the case where the crosslinking reaction is initiated, for example, by addition of the crosslinking accelerator), if the gel composition is applied to the base material too soon after the addition of the crosslinking accelerator, the middle layer gets too thick. On the other hand, if the gel composition is applied to the base material too late, the middle layer cannot be formed, and the absence of the middle layer causes the gel layer to delaminate easily from the base material. Therefore, in this case, the gel composition is applied to the base material preferably within five minutes, or more preferably within one to three minutes, after the start of the crosslinking reaction.

The adhesive surface of the gel layer may be covered by a protective film so as to avoid migration of the cosmetic composition contained in the gel layer. The protective film may be, for example, a film made of polyethylene, polypropylene, polyester, etc.

The thus configured cosmetic gel sheet is packaged separately or as a set of several sheets. The cosmetic gel sheet may be directly wrapped by a film, or may be put in a storage recess of a storage tray and wrapped by a film. However, in the case where the cosmetic gel sheet is directly wrapped by a film, there is a concern that the cosmetic composition may bleed out of the gel layer to the inside of the film package, under an external stress during storage or transport. Further, if the cosmetic composition is subjected to considerable temperature changes during warehouse storage or transport, volatile components evaporate from the gel layer according to the saturated vapor pressure and fill the inside of the film package in a high-temperature environment, whereas the volatile components condense and are absorbed again by the gel layer in a low-temperature environment. While repeating the evaporation and the condensation, the cosmetic gel sheet is feared to deteriorate progressively as the days go by. Therefore, the cosmetic gel sheet is preferably vacuum-packed by being hermetically contained in a depressurized package. Having said that, the degree of mixing due to the bleeding or the evaporation/condensation may be less influential in some kinds of the cosmetic composition. Hence, the packaging can be decided in accordance with the kinds of the cosmetic composition.

[Examples]

Hereinafter, the present invention is described concretely by way of Examples and Comparative Examples, which should not be construed in a limitative manner. To start with, methods for measuring and evaluating various charactersitics of the cosmetic gel sheet are described.

[Method for measuring the elongation percentage in the direction $D_A$ and the elongation percentage in the direction $D_B$]

For the cosmetic gel sheet, the elongation percentage in the direction $D_A$ and the elongation percentage in the direction $D_B$ were measured in the following manner. A first test piece having a width of 50 mm and a length of 60 mm was cut out from the cosmetic gel sheet, with longer sides of the first test piece being aligned in TD directions (directions orthogonal to a machine direction) of the base material of the cosmetic gel sheet. A second test piece having a width of 50 mm and a length of 60 mm was cut out from the cosmetic gel sheet, with longer sides of the second test piece being aligned in the MD direction (the machine direction) of the base material of the cosmetic gel sheet. Each of the first test piece and the second test piece was chucked at an interval of 40 mm, and stretched in longitudinal directions of the respective test pieces at an elongation rate of 80 mm/min. The length L was measured under a load of 1 N. The elongation percentages of the first test piece and the second test piece were calculated, using the following formula.

$$\text{Elongation percentage (\%)} = [(L{-}40)/40] \times 100$$

Of the calculated elongation percentages, the greater one was taken as the elongation percentage in the direction $D_A$, and the smaller one was taken as the elongation percentage in the direction $D_B$.

[Method for measuring the elastic recovery percentage of elongation in the direction $D_A$]

For the cosmetic gel sheet, the elastic recovery percentage of elongation in the direction $D_A$ was measured in the following manner. A test piece having a width of 25 mm and a length of 60 mm was cut out from the cosmetic gel sheet, with longer sides of the first test piece being aligned in the direction $D_A$ of the cosmetic gel sheet determined by the above-described method for measuring the elongation percentages. The test piece was chucked at an interval of 40 mm, and stretched in longitudinal directions of the test piece at an elongation rate of 300 mm/min until the chuck distance reached 60 mm. In this stretched state, the test piece was kept at 23°C, 55% RH for 10 minutes. Thereafter, the chuck distance was narrowed at the same rate as the elongation rate, and the load-elongation curve was obtained. Based on the load-elongation curve, the length of elongation L' when the residual stress was zero was obtained ("the chuck distance under zero residual stress" minus "the chuck distance before measurement (40 mm)"). Using the following formula and the length of elongation L' under zero residual stress, the elastic recovery percentage of elongation in the direction $D_A$ was calculated.

$$\text{Elastic recovery percentage of elongation } (\%)$$
$$= [[(60\text{-}40)\text{-}L']/(60\text{-}40)] \times 100$$

[Method for measuring the vertical adhesion strength]

For the cosmetic gel sheet, the vertical adhesion strength was measured in the following manner. The cosmetic gel sheet was cut into a size of 30 mm × 30 mm. A bottom surface of a 12-mm-diameter cylinder made of stainless steel SUS304 was pressed against the gel layer surface of the cut piece of the cosmetic gel sheet for 10 seconds at a speed of 1 mm/min to give a load of 0.294 N, and removed at a speed of 300 mm/min. The force of removal was measured as the vertical adhesion strength. The thus measured vertical adhesion strength was evaluated by the following three grades.

<Evaluation criteria for the vertical adhesion strength>

| | |
|---|---|
| Excellent (A): | from 0.020 N/12 mmφ (inclusive) to 2.00 N/12 mmφ (inclusive) |
| Good (B): | from 2.00 N/12 mmφ (exclusive) to 5.00 N/12 mmφ (inclusive) |
| Poor (C): | less than 0.020 N/12 mmφ |

[Method for evaluating adhesion property, moisture-retaining property, and skin-firming feel]

The cosmetic gel sheet was processed in the shapes illustrated in Fig. 1 so as to have a longer dimension of 71 mm (a crosswise dimension in Fig. 1) and a shorter dimension of 30 mm (a vertical dimension in Fig. 1), with the longer dimension being aligned in the direction $D_A$. Seven test users applied the processed cosmetic gel sheets to the undereye skin for 15 minutes, and evaluated the adhesion property, the moisture-retaining property, and the skin-firming feel in two or three grades based on the evaluation criteria given below. Of the two- or three-grade evaluations, the most or more common grades chosen by the test users were regarded as final evaluations.

<Evaluation criteria for adhesion property>

| | |
|---|---|
| Good (B): | After the application, the test user felt the cosmetic gel sheet fitting on and following the skin. |
| Poor (C): | After the application, the test user did not feel the cosmetic gel sheet fitting on and following the skin. |

<Evaluation criteria for moisture-retaining property>

| | |
|---|---|
| Good (B): | After the removal of the cosmetic gel sheet, the test user felt the skin moist. |
| Poor (C): | After the removal of the cosmetic gel sheet, the test user did not feel the skin moist. |

<Skin-firming property>

| | |
|---|---|
| Excellent (A): | In the entire part of the skin to which the cosmetic gel sheet was applied, the test user felt that the skin was effectively stretched, and recognized a significant facelift effect. |
| Good (B): | In the entire part of the skin to which the cosmetic gel sheet was applied, the test user felt that the skin was stretched, and recognized a facelift effect. |
| Poor (C): | Despite the application of the cosmetic gel sheet, the test user did not feel that the entire skin was stretched but felt that the skin was stiff. |

[Example 1]

In a glass or plastic first vessel, following ingredients were blended to give a mixture (on all occasions in Examples and Comparative Examples, the unit "% by weight" in parentheses after the unit "part by weight" means "% by weight" relative to 100 % by weight of the gel composition): 69.80 parts by weight (69.80% by weight) of water; 3.5 parts by weight (3.5% by weight) of sodium polyacrylate as the polymer skeleton; 0.8 parts by weight (0.8% by weight) of magnesium aluminometasilicate as the crosslinking agent; 10 parts by weight (10% by weight) of dipropylene glycol as the moisturizer; 2.0 parts by weight (2.0% by weight) of polyethylene glycol 1000 as the bleed accelerator; 1.0 part by weight (1.0% by weight) of glycerol as the moisturizer; 4.0 parts by weight (4.0% by weight) of diglycerol as the moisturizer; 0.3 parts by weight (0.3% by weight) of hydroxypropyl guar gum as the thickening agent; 2.0 parts by weight (2.0% by weight) of olive squalane as the moisturizer; 0.1 part by weight (0.1% by weight) of hydrolyzed collagen as the plant/animal extract; and 0.5 parts by weight (0.5% by weight) of phenoxyethanol as the antiseptic.

Next, in a second vessel, 1.8 parts by weight (1.8% by weight) of tartaric acid as the crosslinking accelerator was dissolved in 4.2 parts by weight (4.2 % by weight) of water to give a tartaric acid aqueous solution. This tartaric acid aqueous solution was added to the mixture in the first vessel to give a gel composition having a pH of 5.

The base material was a nonwoven fabric (trade name "Bemliese® NE107", manufactured by Asahi Kasei Fibers Corporation) made of continuous filaments of regenerated cellulose (cupro) whose raw material was a cotton linter, the fabric being produced by a spunbond or spunlace process and having a mass per unit area of 100 g/m$^2$. The gel composition was applied to this base material. Application of the gel composition was completed within three minutes after the tartaric acid aqueous solution was added to the mixture in the first vessel. The gel composition was covered with a removable 0.10-mm-thick polyester film which served as a protective film, and was cured for 24 hours. Thus obtained was a cosmetic gel sheet which had a gel layer thickness of 0.85 mm and a total thickness (the sum of the thicknesses of the base material, the gel layer, and the removable film) of 1.3 mm.

[Example 2]

The base material was changed from the nonwoven fabric (trade name "Bemliese® NE107", manufactured by Asahi Kasei Fibers Corporation) to a nonwoven fabric having a mass per unit area of 100 g/m$^2$ in which the elongation percentage in the direction $D_A$ was 10%, the elongation percentage in the direction $D_B$ was 1%, and the elastic recovery percentage of elongation in the direction $D_A$ was 0.5%. Except for this change, a cosmetic gel sheet was obtained in the same manner as in Example 1. This cosmetic gel sheet had a gel layer thickness of 0.50 mm and a total thickness of 1.0 mm.

[Example 3]

The base material was changed from the nonwoven fabric (trade name "Bemliese® NE107", manufactured by Asahi Kasei Fibers Corporation) to a nonwoven fabric having a mass per unit area of 80 g/m$^2$ in which the elongation percentage in the direction $D_A$ was 40%, the elongation percentage in the direction $D_B$ was 1%, and the elastic recovery percentage of elongation in the direction $D_A$ was 0.5%. Except for this change, a cosmetic gel sheet was obtained in the same manner as in Example 1. This cosmetic gel sheet had a gel layer thickness of 0.70 mm and a total thickness of 1.1 mm.

[Example 4]

The base material was changed from the nonwoven fabric (trade name "Bemliese® NE107", manufactured by Asahi Kasei Fibers Corporation) to a nonwoven fabric having a mass per unit area of 60 g/m$^2$ in which the elongation percentage in the direction $D_A$ was 30%, the elongation percentage in the direction $D_B$ was 2%, and the elastic recovery percentage of elongation in the direction $D_A$ was 0.5%. Except for this change, a cosmetic gel sheet was obtained in the same manner as in Example 1. This cosmetic gel sheet had a gel layer thickness of 0.80 mm and a total thickness of 1.2 mm.

[Example 5]

The base material was changed from the nonwoven fabric (trade name "Bemliese® NE107", manufactured by Asahi Kasei Fibers Corporation) to a nonwoven fabric having a mass per unit area of 50 g/m$^2$ in which the elongation percentage in the direction $D_A$ was 30%, the elongation percentage in the direction $D_B$ was 1%, and the elastic recovery percentage of elongation in the direction $D_A$ was 5%. Except for this change, a cosmetic gel sheet was obtained in the same manner as in Example 1. This cosmetic gel sheet had a gel layer thickness of 0.80 mm and a total thickness of 1.3 mm.

[Example 6]

Following ingredients were blended and dissolved under stirring to give a monomer solution: 20 parts by weight of acrylamide as the (meth)acrylamide and/or the (meth)acrylamide derivative; 0.2 parts by weight of N,N'-methylenebisacrylamide as the crosslinkable monomer; 45 parts by weight of glycerol as the moisturizer; 5 parts by weight of sodium chloride as the electrolyte; and water, such that the total amount of the ingredients was 100 parts by weight. To 100 parts by weight of this monomer solution, 0.3 parts by weight of 1-hydroxy-cyclohexylphenyl ketone (trade name "IRGACURE® 184", manufactured by BASF Japan Ltd.) as the photopolymerization initiator was added and dissolved under further stirring. The resulting monomer solution was spread thinly on a PET film. The monomer solution was then exposed to ultraviolet irradiation at an intensity of 50 mW/cm$^2$ to effect polymerization crosslinking reaction, thereby giving a sheet-like aqueous gel composition having a thickness of 0.30 mm.

Except for employing this sheet-like aqueous gel composition instead of the gel composition employed in Example 1, a cosmetic gel sheet was obtained in the same manner as in Example 1. This cosmetic gel sheet had a gel layer thickness of 0.30 mm and a total thickness of 0.75 mm.

[Example 7]

The base material was changed from the nonwoven fabric (trade name "Bemliese® NE107", manufactured by Asahi Kasei Fibers Corporation) to a nonwoven fabric having a mass per unit area of 130 g/m$^2$ in which the elongation percentage in the direction $D_A$ was 15%, the elongation percentage in the direction $D_B$ was 1%, and the elastic recovery percentage of elongation in the direction $D_A$ was 3% (trade name "NR91130", manufactured by SANWA SEISHI Co., Ltd). Except for this change, a cosmetic gel sheet was obtained in the same manner as in Example 1. This cosmetic gel sheet had a gel layer thickness of 0.65 mm and a total thickness of 1.1 mm.

[Example 8]

The base material was changed from the nonwoven fabric (trade name "Bemliese® NE107", manufactured by Asahi Kasei Fibers Corporation) to a nonwoven fabric having a mass per unit area of 100 g/m$^2$ in which the elongation percentage in the direction $D_A$ was 15%, the elongation percentage in the direction $D_B$ was 1%, and the elastic recovery percentage of elongation in the direction $D_A$ was 1% (trade name "NM91100", manufactured by SANWA SEISHI Co., Ltd). Except for this change, a cosmetic gel sheet was obtained in the same manner as in Example 1. This cosmetic gel sheet had a gel layer thickness of 0.75 mm and a total thickness of 1.2 mm.

[Example 9]

The base material was changed from the nonwoven fabric (trade name "Bemliese® NE107", manufactured by Asahi Kasei Fibers Corporation) to a nonwoven fabric having a mass per unit area of 120 g/m$^2$ in which the elongation percentage in the direction $D_A$ was 33%, the elongation percentage in the direction $D_B$ was 2%, and the elastic recovery percentage of elongation in the direction $D_A$ was 1% (trade name "NM91120", manufactured by SANWA SEISHI Co., Ltd). Except for this change, a cosmetic gel sheet was obtained in the same manner as in Example 1. This cosmetic gel sheet had a gel layer thickness of 0.70 mm and a total thickness of 1.1 mm.

[Comparative Example 1]

The base material was changed from the nonwoven fabric (trade name "Bemliese® NE107", manufactured by Asahi Kasei Fibers Corporation) to a base material in which the elongation percentages in the directions $D_A$ and $D_B$ were both 0%, and the elastic recovery percentage of elongation in the direction $D_A$ was 0%. Except for this change, a cosmetic gel sheet was obtained in the same manner as in Example 1. This cosmetic gel sheet had a gel layer thickness of 0.70 mm and a total thickness of 1.0 mm.

[Comparative Example 2]

The base material was changed from the nonwoven fabric (trade name "Bemliese® NE107", manufactured by Asahi Kasei Fibers Corporation) to a nonwoven fabric having a mass per unit area of 60 g/m$^2$ in which the elongation percentage in the direction $D_A$ was 30%, the elongation percentage in the direction $D_B$ was 30%, and the elastic recovery percentage of elongation in the direction $D_A$ was 2%. Except for this change, a cosmetic gel sheet was obtained in the same manner as in Example 1. This cosmetic gel sheet had a gel layer thickness of 0.80 mm and a total thickness of 1.2 mm.

[Comparative Example 3]

Following ingredients were blended and dissolved under stirring to give a monomer solution: 20 parts by weight of acrylamide as the (meth)acrylamide and/or the (meth)acrylamide derivative; 0.2 parts by weight of N,N'-methylenebisacrylamide as the crosslinkable monomer; 45 parts by weight of glycerol as the moisturizer; 5 parts by weight of sodium chloride as the electrolyte; 3 parts by weight of polyvinyl alcohol having a viscosity average degree of polymerization of 1800 and a degree of saponification of 88%; and water, such that the total amount of the ingredients was 100 parts by weight. To 100 parts by weight of this monomer solution, 0.3 parts by weight of 1-hydroxy-cyclohexylphenyl ketone (trade name "IRGACURE® 184", manufactured by BASF Japan Ltd.) as the photopolymerization initiator was added and dissolved under further stirring. The resulting monomer solution was spread thinly on a PET film. The monomer solution was then exposed to ultraviolet irradiation at an intensity of 50 mW/cm$^2$ to effect polymerization crosslinking reaction, thereby giving a sheet-like gel composition having a thickness of 0.5 mm.

The base material was a nonwoven fabric (trade name "Bemliese® NE107", manufactured by Asahi Kasei Fibers Corporation) made of continuous filaments of regenerated cellulose (cupro) whose raw material was a cotton linter, the fabric being produced by a spunbond or spunlace process and having a mass per unit area of 100 g/m$^2$. This base material was attached to the sheet-like gel composition to give a cosmetic gel sheet.

This cosmetic gel sheet had a gel layer thickness of 0.50 mm and a total thickness of 0.95 mm.

[Comparative Example 4]

The same nonwoven fabric as the one employed in Example 1 (manufactured by Asahi Kasei Fibers Corporation, trade name "Bemliese ®NE107") having a mass per unit area of 100 g/m$^2$ was employed by itself as a cosmetic gel sheet in Comparative Example 4.

[Comparative Example 5]

The base material was changed from the nonwoven fabric (trade name "Bemliese® NE107", manufactured by Asahi Kasei Fibers Corporation) to a nonwoven fabric having a mass per unit area of 100 g/m$^2$ in which the elongation percentage in the direction $D_A$ was 42%, the elongation percentage in the direction $D_B$ was 12%, and the elastic recovery percentage of elongation in the direction $D_A$ was 4% (trade name "NR91100", manufactured by SANWA SEISHI Co., Ltd). Except for this change, a cosmetic gel sheet was obtained in the same manner as in Example 1. This cosmetic gel sheet had a gel layer thickness of 0.70 mm and a total thickness of 1.2 mm.

[Comparative Example 6]

The base material was changed from the nonwoven fabric (trade name "Bemliese® NE107", manufactured by Asahi Kasei Fibers Corporation) to a nonwoven fabric having a mass per unit area of 50 g/m$^2$ in which the elongation percentage in the direction $D_A$ was 8%, the elongation percentage in the direction $D_B$ was 1%, and the elastic recovery percentage of elongation in the direction $D_A$ was 1% (trade name "NA92050", manufactured by SANWA SEISHI Co., Ltd). Except for this change, a cosmetic gel sheet was obtained in the same manner as in Example 1. This cosmetic gel sheet had a gel layer thickness of 0.80 mm and a total thickness of 1.2 mm.

Regarding the cosmetic gel sheets in these Examples and Comparative Examples, Table 1 shows the measurement results of the elongation percentage in the direction $D_A$, the elongation percentage in the direction $D_B$, the elastic recovery percentage of elongation in the direction $D_A$, and the vertical adhesion strength, as well as the evaluation results of the adhesion property, the moisture-retaining property, and the skin-firming feel, in relation to the mass per unit area of the base material, and the thickness of the gel layer.

[Table 1]

| | | Examples | | | | | | | | | Comparative Examples | | | | | |
|---|---|---|---|---|---|---|---|---|---|---|---|---|---|---|---|---|
| | | 1 | 2 | 3 | 4 | 5 | 6 | 7 | 8 | 9 | 1 | 2 | 3 | 4 | 5 | 6 |
| Mass per unit area of base material (g/m$^2$) | | 100 | 100 | 80 | 60 | 50 | 100 | 130 | 100 | 120 | - | 60 | 100 | 100 | - 100 | 50 |
| Gel layer thickness (mm) | | 0.85 | 0.50 | 0.70 | 0.80 | 0.80 | 0.30 | 0.65 | 0.75 | 0.70 | 0.70 | 0.80 | 0.50 | - | 0.70 | 0.80 |
| Elongation percentage (%) | direction $D_A$ | 30 | 10 | 40 | 30 | 30 | 30 | 15 | 15 | 33 | 0 | 30 | 25 | 35 | 42 | 8 |
| | direction $D_B$ | 1 | 1 | 1 | 2 | 1 | 1 | 1 | 1 | 2 | 0 | 30 | 0.5 | 1.5 | 12 | 1 |
| Elastic recovery percentage of elongation in direction $D_A$ (%) | | 0.5 | 0.5 | 0.5 | 0.5 | 5 | 0.5 | 3 | 1 | 1 | 0 | 2 | 10 | 0.5 | 4 | 1 |
| Vertical adhesion strength (N/12 mmφ) | | A | A | A | A | A | B | A | A | A | A | A | B | C | A | A |
| Adhesion property | | B | B | B | B | B | B | B | B | B | C | B | B | C | B | C |
| Moisture-retaining property | | B | B | B | B | B | B | B | B | B | B | C | C | C | C | B |
| Skin-firming feel | | A | A | A | A | A | B | A | A | A | C | C | C | C | C | C |

A: Excellent
B: Good
C: Poor

As apparent from the results in Table 1, the cosmetic gel sheets in Examples 1 to 9 in which the elongation percentages in the direction $D_A$ were from 10 to 40% and the elongation percentages in the direction $D_B$ were 2% or less (specifically, 1 to 2%) showed better results in adhesion property and skin-firming feel, compared with the cosmetic gel sheets in Comparative Examples 1 and 6 in which the elongation percentages in the direction $D_A$ and the elongation percentages in the direction $D_B$ were both less than 10% (specifically, the elongation percentages in the direction $D_A$ were from 0 to 8%, and the elongation percentages in the direction $D_B$ were from 0 to 1%).

Also as apparent from the results in Table 1, the cosmetic gel sheets in Examples 1 to 9 in which the elongation percentages in the direction $D_A$ were from 10 to 40% and the elongation percentages in the direction $D_B$ were 2% or less (specifically, 1 to 2%) showed better results in moisture-retaining property and skin-firming feel, compared with the cosmetic gel sheets in Comparative Examples 2 and 5 in which the elongation percentages in the direction $D_A$ and the elongation percentages in the direction $D_B$ were both 10% or higher (specifically, the elongation percentages in the direction $D_A$ were from 30 to 42%, and the elongation percentages in the direction $D_B$ were from 12 to 30%).

Also as apparent from the results in Table 1, the cosmetic gel sheets in Examples 1 to 9 in which the elongation percentages in the direction $D_A$ were from 10 to 40% and the elastic recovery percentages of elongation in the direction $D_A$ were 5% or less (specifically, 0.5 to 5%) showed better results in moisture-retaining property and skin-firming feel, compared with the cosmetic gel sheets in Comparative Example 3 in which the elongation percentage in the direction $D_A$ was 10 to 40% and the elastic recovery percentage of elongation in the direction $D_A$ was over 5% (specifically, 10%).

Also as apparent from the results in Table 1, the cosmetic gel sheets in Examples 1 to 9 having the gel layers showed better results in adhesion property, moisture-retaining property and skin-firming feel, compared with the cosmetic gel sheet in Comparative Example 4 having no gel layer.

As described above, the cosmetic gel sheets in Examples 1 to 9 showed a remarkable adhesion property, moisture-retaining property, and skin-firming feel, owing to the gel layers, the elongation percentages in the direction $D_A$ of 10 to 40%, the elastic recovery percentages of elongation in the direction $D_A$ of 5% or less, and the elongation percentages in the direction $D_B$ of 2% or less.

[Reference Signs List]

| 10L, 10R | cosmetic gel sheets |
|---|---|
| 20, 20U, 20L | cosmetic gel sheets |
| 22, 23 | holes |
| 24, 25 | slits |

**Claims**

1. A cosmetic gel sheet comprising a base material made of a nonwoven or woven fabric, and a gel layer containing a cosmetic composition, wherein,
   with a proviso that one direction orthogonal to a thickness direction is a direction $D_A$, and a direction orthogonal to both of the thickness direction and the one direction is a direction $D_B$,
   an elongation percentage in the direction $D_A$ of the cosmetic gel sheet is 10 to 40%,
   an elastic recovery percentage of elongation, measured after the cosmetic gel sheet is stretched in the direction $D_A$ by 50% and kept stretched at 23°C, 55% RH for 10 minutes, is 5% or less, and
   an elongation percentage in the direction $D_B$ of the cosmetic gel sheet is 2% or less.

2. The cosmetic gel sheet according to claim 1,
   wherein the cosmetic gel sheet has a vertical adhesion strength of 0.020 to 5.00 N/12 mmφ.

3. The cosmetic gel sheet according to claim 1 or 2,
   wherein the nonwoven or woven fabric has a mass per unit area of 40 to 150 g/m$^2$.

4. The cosmetic gel sheet according to any one of claims 1 to 3,
   wherein the gel layer has a thickness of 0.3 to 1.5 mm.

5. The cosmetic gel sheet according to any one of claims 1 to 4,
   wherein the gel layer is a hydrogel layer containing 25 to 95% by weight of water.

6. The cosmetic gel sheet according to any one of claims 1 to 5, wherein the cosmetic gel sheet is used for application to a face.

FIG.1

FIG.2

FIG.3

FIG.4

## INTERNATIONAL SEARCH REPORT

| International application No. |
| --- |
| PCT/JP2015/077816 |

### A. CLASSIFICATION OF SUBJECT MATTER
*A45D44/22*(2006.01)i, *A61K8/02*(2006.01)i, *A61K8/26*(2006.01)i, *A61K8/73*
(2006.01)i, *A61K8/81*(2006.01)i, *A61Q19/00*(2006.01)i

According to International Patent Classification (IPC) or to both national classification and IPC

### B. FIELDS SEARCHED

Minimum documentation searched (classification system followed by classification symbols)
A45D44/22, A61K8/00-8/99, A61Q19/00

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2001-58924 A (Kao Corp.), 06 March 2001 (06.03.2001), paragraphs [0012], [0027] (Family: none) | 1-6 |
| A | JP 2010-281003 A (Daiwabo Holdings Co., Ltd.), 16 December 2010 (16.12.2010), paragraphs [0007], [0021] (Family: none) | 1-6 |
| A | WO 2006/016601 A1 (Daiwabo Co., Ltd.), 16 February 2006 (16.02.2006), paragraphs [0061], [0063], [0065] & US 2008/0069845 A1 paragraphs [0065] to [0066], [0068] & EP 1813167 A1 & KR 10-2007-0052754 A & CN 101010017 A | 1-6 |

[X] Further documents are listed in the continuation of Box C.　　　[ ] See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 17 December 2015 (17.12.15) | 28 December 2015 (28.12.15) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (July 2009)

**INTERNATIONAL SEARCH REPORT**

International application No.

PCT/JP2015/077816

C (Continuation). DOCUMENTS CONSIDERED TO BE RELEVANT

| Category* | Citation of document, with indication, where appropriate, of the relevant passages | Relevant to claim No. |
|---|---|---|
| A | JP 2013-226179 A (Asahi Kasei Fibers Corp.), 07 November 2013 (07.11.2013), paragraphs [0002], [0027], [0030] (Family: none) | 1-6 |
| A | JP 2010-222320 A (Sekisui Plastics Co., Ltd.), 07 October 2010 (07.10.2010), paragraphs [0001], [0034] & CN 101843567 A & KR 10-2010-0107419 A | 1-6 |
| A | WO 2013/108918 A1 (Sekisui Plastics Co., Ltd.), 25 July 2013 (25.07.2013), paragraph [0062] (Family: none) | 1-6 |
| A | JP 2011-178693 A (Kose Corp.), 15 September 2011 (15.09.2011), paragraph [0044] (Family: none) | 1-6 |
| A | JP 2000-143484 A (Nitto Denko Corp.), 23 May 2000 (23.05.2000), paragraphs [0016], [0033], [0036] (Family: none) | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013128743 A

- JP 2000128732 A